(19)

Europäisches
Patentamt
European
Patent Office
Office européen
des brevets

(11)  **EP 2 351 734 B1**

(12)  **EUROPEAN PATENT SPECIFICATION**

(45) Date of publication and mention
of the grant of the patent:
**12.03.2014 Bulletin 2014/11**

(21) Application number: **09823712.6**

(22) Date of filing: **26.10.2009**

(51) Int Cl.:
*C07C 303/24* (2006.01)    *C07C 305/04* (2006.01)
*C08G 65/324* (2006.01)    *C08G 65/326* (2006.01)

(86) International application number:
**PCT/JP2009/068732**

(87) International publication number:
**WO 2010/050602 (06.05.2010 Gazette 2010/18)**

(54) **PROCESS FOR PRODUCING SULFURIC ACID ESTER SALT**

HERSTELLUNGSVERFAHREN FÜR SCHWEFELSÄUREESTERSALZ

PROCÉDÉ DE FABRICATION D'UN SEL D'ESTER DE L'ACIDE SULFURIQUE

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **27.10.2008 JP 2008275163**

(43) Date of publication of application:
**03.08.2011 Bulletin 2011/31**

(73) Proprietor: **Kao Corporation
Chuo-ku
Tokyo 103-8210 (JP)**

(72) Inventors:
• **TOMIFUJI, Takeshi
Wakayama-shi
Wakayama 640-8580 (JP)**
• **MITSUDA, Yoshinori
Wakayama-shi
Wakayama 640-8580 (JP)**

• **CHIBA, Keisuke
Wakayama-shi
Wakayama 640-8580 (JP)**
• **FUKUSHIMA, Tetsuaki
Wakayama-shi
Wakayama 640-8580 (JP)**
• **KONO, Jun
Wakayama-shi
Wakayama 640-8580 (JP)**

(74) Representative: **HOFFMANN EITLE
Patent- und Rechtsanwälte
Arabellastrasse 4
81925 München (DE)**

(56) References cited:
**JP-A- 7 157 464      JP-A- 11 315 061
JP-A- 2000 038 374    JP-B- 38 018 658**

Remarks:
The file contains technical information submitted after
the application was filed and not included in this
specification

EP 2 351 734 B1

**Description**

Field of the invention

The present invention relates to a method for producing a sulfate having an oxyalkylene group.

Background of the invention

Polyoxyalkylene alkyl ether sulfates have been conventionally used as a surfactant. These are produced by reacting alkoxylate compounds, which are raw materials, with $SO_3$ gas to sulfate, and neutralizing the sulfated products.

Sulfation of an alkoxylate compound as a raw material with $SO_3$ gas is a highly exothermic reaction, and has limitation in reaction temperature due to stability of a sulfated product. In such conditions, the reaction product is viscous and has a low fluidity and easily reacts with $SO_3$ in excess. There are thus problems such as coloration of the product or generation of odoriferous side products such as carbonized and cyclized substances by decomposition due to over-reactions. In some cases, particularly in various commercial uses, products having bad color and/or odor may reduce their commodity values. To counter such problems including bad color and odor, there have been proposed many methods.

JP-B49-48409 discloses a method of sulfation with a thin film sulfation apparatus by introducing the air or an inert gas as a cooling gas between a thin film flow of reaction liquid and an $SO_3$ gas in an amount of about 2 to 5 molar of the $SO_3$ gas at substantially the same rate as the $SO_3$ gas flow while keeping a reaction temperature at a constant level to give a sulfated product little colored with a small amount of side products. However, the method of sulfation uses a large amount of inert gas for cooling, and requires measures for recycling an exhaust gas as described in JP-B53-41130 filed by the same applicant as JP-B49-48409 at a later date. The method thus has a disadvantage of large equipment load. In addition, the method of sulfation has little effect of improving a color of a sulfated product, and must be further improved.

JP-A7-157464 discloses a method of treating an aqueous slurry containing a polyoxypropylene alkyl ether sulfate at a specific concentration with steam under specific conditions, thereby enabling effective deodorization. JP-A2000-38374 discloses a method for producing a sulfated product with markedly improved color at low cost by supplying an $SO_3$ gas and an inert gas together within a specific range. JP-A11-315061 (JP-B2995403) discloses a sulfate producing apparatus including a starting compound feeder having an overflow mechanism that enables feeding the starting compound to the inner wall of a reaction tube by its overflowing.

JP-A2001-151746 and JP-A2001-187776 disclose methods for producing a polyoxyethylene alkyl ether sulfate, suppressing 1,4-dioxane from generating as a side product in the product mixture by specifying temperatures for reaction and treatment and residence time in a reaction tube.

US-B6017875 discloses sulfation of an alkylene oxide adduct of a secondary alcohol.

Summary of the invention

The present invention relates to a method for producing a sulfate represented by the formula (2), including steps of:

feeding a compound represented by the formula (1) (starting compound) as a flow running down along an inner wall of a reaction tube to form a thin film of the starting compound on the inner wall of the reaction tube, the tube being provided in a jacket system containing two or more temperature-controlled jackets in which a temperature-controlling fluid can flow, and together feeding $SO_3$ gas into the reaction tube to react the starting compound with the $SO_3$ gas to obtain a sulfated product (hereinafter referred to as sulfation step); and neutralizing the resultant sulfated product (hereinafter referred to as neutralizing step), wherein the two or more jackets include an upstream jacket placed on an upstream part and a downstream jacket placed on a downstream part in a direction of the flow of the starting compound, an exit temperature of the temperature-controlling fluid flowing in the upstream jacket is not less than 35°C and not more than 70°C, an exit temperature of the temperature-controlling fluid flowing in the downstream jacket is not less than 10°C and not more than 30°C, a length of the upstream jacket is 40 to 60% of the whole length of the reaction tube, and a length of the downstream jacket is 60 to 40% of the whole length of the reaction tube:

$$\text{R-O-} (CH_2CH_2O)_p\text{-} (C_nH_{2n}O)_m\text{-} (CH_2CH_2O)_k\text{-H} \qquad (1)$$

where R represents a hydrocarbon group having 8 to 22 carbon atoms; p and k each represents a number, a summation average of p and k being not less than 0 and not more than 40; n represents a number of 3 to 4; and m represents a number of more than 0 and not more than 5 on average,

$$R\text{-}O\text{-}(CH_2CH_2O)_p\text{-}(C_nH_{2n}O)_m\text{-}(CH_2CH_2O)_k\text{-}SO_3M \qquad (2)$$

where R represents a hydrocarbon group having 8 to 22 carbon atoms; p and k each represents a number, a summation average of p and k being not less than 0 and not more than 40; n represents a number of 3 to 4; m represents a number of more than 0 and not more than 5 on average; M represents an alkaline metal ion, an alkaline earth metal ion, an ammonium ion, or mono-, di-, or trialkanolammonium ion having an alkanol group having 2 to 3 carbon atoms.

Detailed description of the invention

As described in JP-A7-157464, for a sulfate having an oxypropylene group, a problem of odor cannot be sufficiently solved by the way of dealing with a sulfate having only an oxyethylene group. Other methods are conducted under complex controls. Therefore, there is a need for further study to improve odor of a sulfate having an oxypropylene group. Also for color of the sulfate having an oxypropylene group, there is a need for study from a different point of view from conventional findings.

The present invention relates to providing a method for producing a sulfate having an oxypropylene group, which can produce a reaction product having markedly improved color and/or odor at low cost. As used herein, the "sulfation" refers to a reaction of producing sulfuric acid esters.

According to the present invention, there is provided a method of producing a sulfate having an oxypropylene group, which method can produce a reaction product having markedly improved color and/or odor at low cost. The reaction product is useful as a surfactant.

A starting compound used in the present invention is represented by the formula (1) :

$$R\text{-}O\text{-}(CH_2CH_2O)_p\text{-}(C_nH_{2n}O)_m\text{-}(CH_2CH_2O)_k\text{-}H \qquad (1)$$

where R represents a hydrocarbon group having 8 to 22 carbon atoms; p and k each represents the number, the summation average of p and k is not less than 0 and not more than 40; n represents the number of 3 to 4; and m represents the number not less than 0 and not more than 5 on average.

The compound represented by the formula (1) is not specifically limited. Examples thereof include a block adduct of propylene oxide (hereinafter, abbreviated as PO) and ethylene oxide (hereinafter, abbreviated as EO) to an aliphatic alcohol or an alkylphenol, a block adduct of butylene oxide and ethylene oxide to an aliphatic alcohol or an alkylphenol, a PO-adduct of an aliphatic alcohol and a PO-adduct to an alkylphenol.

In the formula (1), R is preferably an alkyl group. The number of carbon atoms of R is preferably 10 to 18, and more preferably 12 to 14. p and k each represent the number not less than 0. When p=0, the summation average of p and k is preferably more than 0, more preferably not less than 0.7, and even more preferably not less than 1.2, and also preferably not more than 15, more preferably not more than 5, and even more preferably not more than 3. When p≠0, the summation average is preferably not less than 5 and not more than 25, more preferably not less than 8 and not more than 20. In this case, k may or may not be equal to 0. When p+k=0, m is preferably not less than 0.5 and not more than 3. When p≥0 and k≠0, m is preferably not less than 0.2, more preferably not less than 0.3, and even more preferably not less than 0.4, and also preferably not more than 2.5, more preferably not more than 1.5, even more preferably not more than 0.8, and even more preferably not more than 0.6. n represents the number of 3 or 4, preferably 3. A ratio of p+k to k is preferably (p+k)/k≤3.5 (with the proviso that k≠0), more preferably not more than 3, even more preferably not more than 2, and even more preferably not more than 1. When p+k≠0, a ratio of p+k to m, (p+k)/m, is preferably not less than 1 and not more than 20, more preferably not less than 1 and not more than 5, and even more preferably not less than 1 and not more than 4. When k=0, p is preferably more than 0 and not more than 22, and more preferably not less than 10 and not more than 20.

Brief description of the drawings

Fig. 1 shows a schematic diagram of an embodiment of a sulfation reactor used in the present invention.
Fig. 2 shows a schematic diagram of an embodiment of a structure of feed ports of a starting compound, an inert gas, and an $SO_3$ gas in the sulfation reactor in Fig. 1.

In Figures, reference numerals refer to the followings:

1    sulfation reactor
2    starting compound feed port
3    inert gas feed port
4    $SO_3$ gas feed port
5    reaction tube
6    upstream (first) jacket (cooling device)
6'   downstream (second) jacket (cooling device)
7    pot

<Sulfation step>

In the present invention, a sulfate is produced by feeding a starting compound as a flow running down along the inner wall of a reaction tube to form a thin film of the starting compound on the inner wall of the reaction tube, the tube being arranged in a jacket system including two or more temperature-controlled jackets in which temperature-controlling fluid can flow, and feeding $SO_3$ gas to the reaction tube to react the starting compound with the $SO_3$ gas. Two or more jackets include at least an upstream jacket and a downstream jacket. The compound represented by the formula (2) is a sulfate having a structure corresponding to that of the compound represented by the formula (1) used as a raw material.

The reaction tube used in the present invention is not specifically limited. It is preferably a falling film type continuous reactor that can form a thin film of the starting compound represented by the formula (1) on the inner wall thereof by flowing down the compound on the wall and can feed the $SO_3$ gas and the inert gas such that the inert gas flows between the thin film of the starting compound and the $SO_3$ gas flow. Examples of the reactor include those described in Chemithon, Ballestra et al., Kirk Othmer, Encyclopedia of Chemical Technology, 3rd Ed., Wiley, 1983, Vol. 22, pp 28-45, "Sulfonation and Sulfation", W. H. De Groot, "Sulphonation Technology in the Detergent Industry", Kluwer Academic Publishers, Boston, 1991; ISBN 0-7923-1202-3, "Detergent Manufacturing Including Zeolite Builders and Other New Materials", Chemical Technology Review No. 128, Noyes Data Corp., Park Ridge, New Jersey, 1979, pp. 273-310, US-A3931273 (1976), and JP-A2001-181215. In terms of ease of production and availability of equipment, the reaction tube is preferably of a pipe form. It is noted that the number of the reaction tubes used in the reaction is not specifically limited.

From the viewpoint of prevention of pressure drop to reduce energy loss, an inner diameter of the reaction tube, particularly that of a pipe form, is preferably not less than 8 mm, and more preferably not less than 12 mm. From the viewpoint of shortening a pipe length required for completing the reaction to reduce device cost, the inner diameter is preferably not more than 80 mm, more preferably not more than 40 mm, and even more preferably not more than 30 mm. To put it all together, the inner diameter of the reaction tube is preferably 8 to 80 mm, and more preferably 8 to 40 mm. From the viewpoints of completing the reaction to reduce unreacted raw materials and effectively removing reaction heat, a length of the reaction tube, particularly that of a pipe form, is preferably not less than 1 m, more preferably not less than 2 m, and even more preferably not less than 3 m. From the viewpoint of decreasing pressure drop to reduce device cost, the length is preferably not more than 20 m, and more preferably not more than 10 m. To put it all together, the length of the reaction tube is preferably 1 to 15 m, and more preferably 2 to 10 m.

A material for the reaction tube is not specifically limited. Examples of the material include, but are not limited to, stainless steel, glass, fluorine resin, titanium, and other alloys.

The reaction tube may be arranged in any configuration as long as the starting compound can run down the inner wall of the reaction tube. For example, the reaction tube is preferably set in the vertical direction to a horizontal plane.

A flow rate of the starting compound, which may be varied depending on a molecular weight of the starting compound represented by the formula (1), is preferably a flow rate per wetted perimeter of not less than 50 kg/mHr, and more preferably not less than 100 kg/mHr, from the viewpoint of formation of a uniform liquid film. From the viewpoint of prevention of over reaction due to inadequate mixing in a liquid film, the flow rate is preferably a flow rate per wetted perimeter of not more than 800 kg/mHr, and more preferably not more than 600 kg/mHr (e.g., 50 to 800 kg/mHr, and preferably 100 to 600 kg/mHr). For example, when a pipe having an inner diameter of not more than 20 mm such as 14 mm is used as the reaction tube, the flow rate of the starting compound is preferably not less than 2.2 kg/Hr, and more preferably not less than 4.4 kg/Hr, and also preferably not more than 35 kg/Hr, more preferably not more than 26 kg/Hr, even more preferably not more than 17 kg/Hr, and even more preferably not more than 8 kg/Hr (e.g., 2.2 to 35 kg/Hr, and preferably 4.4 to 26 kg/Hr). When a pipe having an inner diameter of more than 20 mm such as 28 mm is used as the reaction tube, the flow rate is preferably not less than 4.4 kg/Hr, and more preferably not less than 8.8 kg/Hr, and also preferably not more than 70 kg/Hr, more preferably not more than 53 kg/Hr, even more preferably not more than 36 kg/Hr, and even more preferably not more than 18 kg/Hr (e.g., 4.4 to 70 kg/Hr, and preferably 8.8 to 53 kg/Hr). A flow rate per wetted perimeter is determined by the following formula:

$$\text{Flow rate per wetted perimeter (kg/mHr)} =$$

$$\text{(flow rate of the starting compound (kg/Hr))}/$$

$$\pi\times\text{(inner diameter of the reaction tube (m))}\times\text{(the}$$

number of the reaction tube)

A feeding temperature of the starting compound represented by the formula (1) to the reactor is only required to be not lower than a melting point or a turbidity point. The starting compound is preferably fed at a steady temperature controlled by a general means. The feeding temperature is preferably not lower than 35°C and not higher than 70°C.

In the present invention, $SO_3$ gas is diluted to an appropriate concentration with the air, nitrogen, or other inert gas to be introduced to the reaction tube. In this case, a concentration of $SO_3$ is generally not less than 1.0% by volume and not more than 10% by volume (v/v), and preferably not less than 2.5% by volume and not more than 6.5% by volume (v/v).

A flow rate of only $SO_3$ gas may be varied depending on an inner diameter of the reaction tube. For example, when the reaction tube has an inner diameter of not more than 20 mm such as 14 mm, from the viewpoint of good progress of the reaction, the flow rate is preferably not less than $1.8\times10^{-3}$ $Nm^3/s$, and more preferably not less than $2.7\times10^{-3}$ $Nm^3/s$. From the viewpoint of decreasing pressure loss to reduce device cost, the flow rate is preferably not more than $9.5\times10^{-3}$ $Nm^3/s$, and more preferably not more than $8.0\times10^{-3}$ $Nm^3/s$ (e.g., $1.8\times10^{-3}$ to $9.5\times10^{-3}$ $Nm^3/s$, and preferably $2.7\times10^{-3}$ to $8.0\times10^{-3}$ $Nm^3/s$). When the reaction tube has an inner diameter of more than 20 mm such as 28 mm, the flow rate is preferably not less than $11\times10^{-3}$ $Nm^3/s$, and more preferably not less than $15\times10^{-3}$ $Nm^3/s$, and also preferably not more than $57\times10^{-3}$ $Nm^3/s$, and more preferably not more than $48\times10^{-3}$ $Nm^3/s$ (e.g., $11\times10^{-3}$ to $57\times10^{-3}$ $Nm^3/s$, and preferably $15\times10^{-3}$ to $48\times10^{-3}$ $Nm^3/s$).

From the viewpoint of formation of annular flow in the reaction to give a uniform liquid film, a flow velocity of $SO_3$ gas is preferably not less than 30 Nm/s, and more preferably not less than 40 Nm/s. From the viewpoint of prevention of disarray of a liquid film and prevention of generation of mist in the reaction, the flow velocity is preferably not more than 150 Nm/s and more preferably not more than 125 Nm/s.

A flow velocity of $SO_3$ gas can be calculated according to a method of calculation described in paragraphs 0036 to 0047 of JP-B3002779.

In the present invention, the flow velocity is preferably controlled such that a molar ratio of the starting compound to the $SO_3$ gas in the reaction is, represented by $SO_3$/starting compound, preferably 0.90 to 1.09, more preferably 0.94 to 1.05, and even more preferably 0.97 to 1.02. When the molar ratio is not less than 0.90, the reaction progresses at good reaction rate with small amount of side products generated. When not more than 1.09, a reaction rate is held at good level.

In the present invention, an inert gas is preferably fed such that the inert gas flows between a thin film of the starting compound and the $SO_3$ gas. The inert gas fed between the starting compound and the $SO_3$ gas is not specifically limited. For example, a gas that chemically reacts with neither the $SO_3$ gas nor the starting compound can be used, including air, dehumidified air, and nitrogen. From the point of cost, the air is preferred. For reducing side products such as sodium sulfate, the dehumidified air is more preferred. A part of the inert gas used for diluting the $SO_3$ gas can also be used without problem. An $SO_3$-containing gas preferably has a concentration of $SO_3$ of 1 to 30% by volume, and more preferably 1 to 20% by volume diluted with an inert gas such as the dehumidified air or nitrogen. A gas having a concentration of $SO_3$ of less than 1% by volume has large gas volume, and a gas having a concentration of $SO_3$ of over 30% by volume may cause overreaction.

At this time, a flow rate may be varied depending on an inner diameter of the reaction tube. For example, when one reaction tube having an inner diameter of not more than 20 mm such as 14 mm is used, the flow rate is preferably not less than $9.0\times10^{-5}$ $Nm^3/s$, and more preferably not less than $1.4\times10^{-4}$ $Nm^3/s$, from the viewpoint of achieving sufficient effects for improving color. From the viewpoint of facilitating the reaction to sufficiently reduce unreacted raw materials, the flow rate is also preferably not more than $6.3\times10^{-3}$ $Nm^3/s$, and more preferably not more than $5.4\times10^{-3}$ $Nm^3/s$ (e.g., $9.0\times10^{-5}$ to $6.3\times10^{-3}$ $Nm^3/s$, and preferably $1.4\times10^{-4}$ to $5.4\times10^{-3}$ $Nm^3/s$). When one reaction tube having an inner diameter of more than 20 mm such as 28 mm is used, the flow rate is preferably not less than $1.1\times10^{-3}$ $Nm^3/s$, and more preferably not less than $1.5\times10^{-3}$ $Nm^3/s$, and also preferably not more than $5.7\times10^{-2}$ $Nm^3/s$, and more preferably not more than $4.8\times10^{-2}$ $Nm^3/s$ (e.g., $1.1\times10^{-3}$ to $5.7\times10^{-2}$ $Nm^3/s$, and preferably $1.5\times10^{-3}$ to $4.8\times10^{-2}$ $Nm^3/s$).

A flow velocity of the inert gas flowing on the starting compound running down the inner wall of the reaction tube may be varied depending on a flow velocity of the $SO_3$ gas flowing through the center of the tube. For example, when one reaction tube having an inner diameter of not more than 20 mm such as 14 mm is used, the flow velocity is preferably not less than 9 Nm/s, and more preferably not less than 12 Nm/s, from the viewpoint of formation of annular flow to give a uniform liquid film. From the viewpoint of prevention of disarray of a liquid film and prevention of generation of mist, the flow velocity is also preferably not more than 180 Nm/s, and more preferably not more than 150 Nm/s (e.g., 9 to 180 Nm/s, and preferably 12 to 150 Nm/s). When one reaction tube having an inner diameter of more than 20 mm such as 28 mm is used, the flow velocity is preferably not less than 3 Nm/s, and more preferably not less than 4 Nm/s, and also preferably not more than 120 Nm/s, and more preferably not more than 100 Nm/s (e.g., 3 to 120 Nm/s, and preferably 4 to 100 Nm/s).

A flow velocity of the inert gas can be similarly calculated according to the method of calculation described in paragraphs 0036 to 0047 of JP-B3002779 as for the flow velocity of the $SO_3$ gas.

A flow ratio of the inert gas to the $SO_3$ gas may be varied depending on flow areas of the $SO_3$ gas and the inert gas. However, increased flow area of the inert gas accompanies increased flow of the inert gas, resulting in excessively suppressed generation of reaction heat and slowing in progress of the reaction. In such a situation, the reaction often does not complete in the reaction tube. Therefore, extremely increased flow area is unfavorable. In contrast, a remarkably decreased flow area accompanies an increased loss of pressure in the flow channel, resulting in a large difference in liquid film's thickness between in the flow channel of the inert gas and after the reaction started. Such a situation induces disarray of the liquid film to an unfavorably decreased production efficiency. From these viewpoints the flow ratio (inert gas/$SO_3$ gas, volume ratio), which is varied depending on an inner diameter of the reaction tube, is preferably not less than 0.01, more preferably not less than 0.015, and even more preferably not less than 0.02, and also preferably not more than 1.8, more preferably not more than 0.5, and even more preferably not more than 0.4. To put it all together, the flow ratio is preferably 0.015 to 0.5, and more preferably 0.02 to 0.4.

Particularly when the reaction tube has an inner diameter of not more than 20 mm, the flow ratio is preferably not less than 0.05, more preferably not less than 0.1, and even more preferably not less than 0.15, and also preferably not more than 1.8, more preferably not more than 0.5, and even more preferably not more than 0.4. To put it all together, the flow ratio is preferably 0.05 to 1.8, more preferably 0.1 to 0.5, and even more preferably 0.15 to 0.4.

Particularly when the reaction tube has an inner diameter of more than 20 mm, the flow ratio is preferably not less than 0.01, more preferably not less than 0.015, and even more preferably not less than 0.02, and also preferably not more than 0.2, more preferably not more than 0.15, and even more preferably not more than 0.1. To put it all together, the flow ratio is preferably 0.01 to 0.2, more preferably 0.015 to 0.15, and even more preferably 0.02 to 0.1.

A flow velocity ratio of the inert gas to the $SO_3$ gas, which may be varied depending on an inner diameter of the reaction tube, is preferably not less than 0.1, more preferably not less than 0.15, and even more preferably not less than 0.2, and also preferably not more than 1.2, more preferably not more than 1.0, and even more preferably not more than 0.9. To put it all together, the flow velocity ratio is preferably 0.15 to 1.0, and more preferably 0.2 to 0.9.

Particularly when the reaction tube has an inner diameter of not more than 20 mm, the flow velocity ratio is preferably not less than 0.3, more preferably not less than 0.4, and even more preferably not less than 0.5, and also preferably not more than 1.2, more preferably not more than 1.0, and even more preferably not more than 0.9. To put it all together, the flow velocity ratio is preferably 0.3 to 1.2, more preferably 0.4 to 1.0, and even more preferably 0.5 to 0.9.

Particularly when the reaction tube has an inner diameter of more than 20 mm, the flow velocity ratio is preferably not less than 0.1, more preferably not less than 0.15, and even more preferably not less than 0.2, and also preferably not more than 0.8, more preferably not more than 0.6, and even more preferably not more than 0.48. To put it all together, the flow velocity ratio is preferably 0.1 to 0.8, more preferably 0.15 to 0.6, and even more preferably 0.2 to 0.48.

In these ranges of the flow velocity ratio, effects of improving color of the resultant sulfated product, particularly the sulfate, are more enhanced. It is thought that in these ranges of the flow velocity ratio, ingress of $SO_3$ gas molecules to the inert gas at the interface between the $SO_3$ gas and the inert gas is controlled to decrease a rate of the $SO_3$ gas molecule to reach the surface of the liquid film of the starting compound, whereby rapid progress of the reaction at the top of the reactor is relaxed and topical heat generation and rapid increase of a reactant temperature are controlled. Further, by controlling the flow velocity ratio within these ranges, a used amount of the inert gas to an amount of the $SO_3$ gas can be decreased from that conventionally used, the method of the present invention does not put load on facilities, and better effect of improving color can be achieved.

The method of the present invention includes a step of sulfation by feeding the starting compound as a flow running down the inner wall of the reaction tube to form a thin film of the starting compound on the inner wall of the reaction tube, the tube being arranged in a jacket system including two or more temperature-controlled jackets in which a temperature-controlling fluid can flow (these two or more jackets include at least the upstream jacket placed on the upstream part and the downstream jacket placed on the downstream part in the direction of the flow of the starting material), and feeding the $SO_3$ gas into the reaction tube to react the starting compound with the $SO_3$ gas to give a sulfated product.

In the step of sulfation, any known thin film type sulfation reactor can be used. In the present invention, used is a reactor having a jacket system including two or more temperature-controlled jackets in which a temperature-controlling fluid can flow, the two or more jackets including the upstream jacket placed on the upstream part and the downstream jacket placed on the downstream part in the direction of the flow of the starting material. From the viewpoint of color of the sulfate, an exit temperature of the temperature-controlling fluid circulated in the upstream jacket is not less than 35°C and not more than 70°C, and preferably not less than 40°C and not more than 55°C. From the viewpoint of odor of the sulfate, an exit temperature of the temperature-controlling fluid circulated in the downstream jacket is not less than 10°C and not more than 30°C, and preferably not less than 10°C and not more than 25°C. From the viewpoint of relaxing heat generation to control reactivity, a length of the upstream jacket is 40 to 60% of the whole length of the reaction tube. From the viewpoint of control of over reaction to improve odor and color, a length of the downstream jacket is 60 to 40% of the whole length of the reaction tube. The other constructions can be similar to known equipments. In the present invention, the jacket system must include at least two jackets (i.e., including just the upstream and downstream jackets). In this case, that is, when the system includes just the upstream and downstream jackets, the total length of the upstream and downstream jackets is preferably 80 to 100%, and more preferably 90 to 100% of the whole length of the reaction tube.

When the jacket system includes three or more jackets, jacket(s) other than the upstream and downstream jackets (other jacket(s)) are preferably placed between the upstream and downstream jackets. However, the other jacket can be placed at any position as long as it does not overlap with the upstream jacket or the downstream jacket. An exit temperature of the temperature-controlling fluid circulated in the other jacket is in the range of exit temperatures of the temperature-controlling fluids circulated in the upstream and downstream jackets, respectively.

In the present invention, among two or more jackets, a top jacket placed on the most upstream part in the direction of the flow of the starting compound may be the upstream jacket, and a bottom jacket placed on the most downstream part may be the downstream jacket.

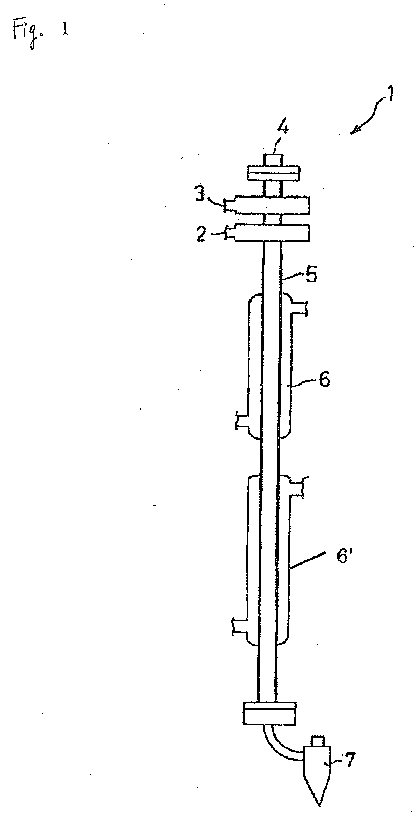

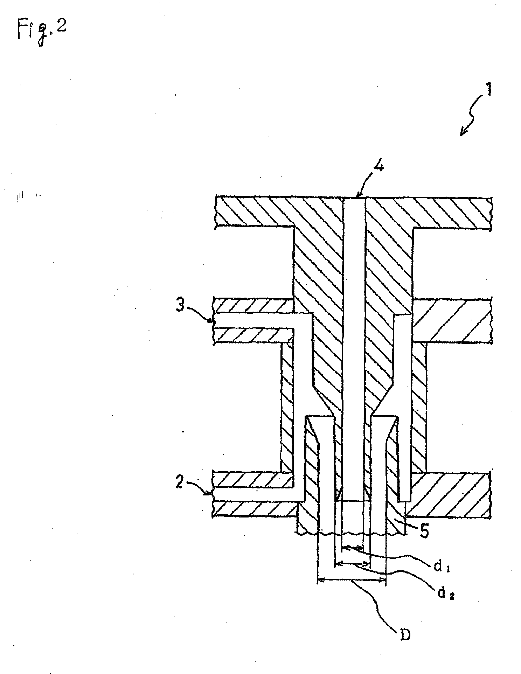

For example, the thin film type sulfation reactor used in the present invention includes a reaction tube that brings a thin film flow of the starting compound into contact with $SO_3$ gas flow in the same direction to react them, a starting compound's feed part to feed the starting compound to the inner wall of the reaction tube, an $SO_3$ gas' feed part to feed the $SO_3$ gas in the reaction tube, and an inert gas feed part to feed the inert gas between the thin film flow and the $SO_3$ gas flow according to need, in which the reaction tube is provided in a jacket system including two jacket in which a temperature-controlling fluid can flow. An example of such a thin film type sulfation reactor is shown in Figs. 1 and 2.

Fig. 1 shows a schematic diagram of a preferred embodiment of the thin-film type sulfation reactor used in the present invention. A sulfation reactor 1 includes a reaction tube 5 having a starting compound feed port 2, an inert gas feed port 3, and an $SO_3$ gas feed port 4. The reaction tube 5 is surrounded by an upper jacket 6 and a lower jacket 6' as shown in Fig. 1. The reactor has two jackets, a first jacket and a second jacket corresponding to the upstream and downstream jackets, respectively, of the present invention.

The starting compound, the inert gas, and the $SO_3$ gas are fed in the reaction tube 5 from the upper part of the reaction tube 5 through the respective feed ports. The starting compound runs down the inner wall of the reaction tube 5 and reacts with the $SO_3$ gas. The reaction tube 5 has a structure that can be externally cooled with the jackets 6 and 6'. To the jackets 6 and 6' is fed cooling water. The reaction tube 5 has a pot 7 to receive a reaction product at the bottom of the tube. The reaction product is divided into a sulfated product and an exhaust gas with a gas-liquid separation device (cyclone).

Fig. 2 shows a schematic diagram of a structure of feed ports of the starting material, the inert gas, and the $SO_3$ gas in the sulfate reactor 1 shown in Fig. 1.

In carrying out the present invention in an industrial scale, a multi-tube reactor having a plurality of reaction tubes incorporated is preferably used. In this case, tubes having inner diameters and lengths in ranges described above can be used for the reaction tubes. The tubes preferably have the same inner diameter and the same length.

For two jackets, in the first jacket 6 placed on the upstream part of the starting compound flow, a temperature-controlling fluid of not less than 35°C and not more than 70°C, preferably not less than 40°C and not more than 55°C flows. In the second jacket 6' placed on the downstream part of the starting compound flow, a temperature-controlling fluid of not less than 10°C and not more than 30°C, preferably not less than 10°C and not more than 20°C flows.

A length of the first jacket 6 is 40 to 60% of the whole length of the reaction tube 5. A length of the second jacket 6' is 60 to 40% thereof.

<Step of neutralization >

The step of neutralizing an alkylsulfuric acid or an alkylalkoxylated sulfuric acid produced in the sulfation step is preferably performed immediately after the sulfation. In general conditions for neutralization, a neutralizing temperature is 20°C to 60°C, and preferably 25°C to 50°C.

Neutralization is conducted with a basic neutralizer. Examples of the basic neutralizer include alkaline metal hydroxides, alkaline earth metal hydroxides, ammonia, and mono-, di-, and trialkanolamines having an alkanol group of 2 to 3 carbon atoms. The basic neutralizer is preferably selected from potassium hydroxide, sodium hydroxide, ammonia, monoethanolamine, and triethanolamine. The final alkylsulfate prepared by the step of neutralization is generally a paste composition containing 20% by mass or more of active component. A concentration of the active component can be appropriately controlled.

The starting compound represented by the formula (1) is produced by adding EO and PO to an alcohol having 8 to 22 carbon atoms in the presence of a catalyst by a known technique. The alcohol may be a natural or synthetic product. The natural or synthetic alcohol may be a single composition of a single carbon number or a mixture of those having different carbon numbers prepared by fractional distillation. Examples of the alcohol include synthetic primary alcohols such as Dobanol (trade name, manufactured by Mitsubishi Chemical Corporation), Diadol (trade name, manufactured by Mitsubishi Chemical Corporation), Oxocol (trade name, manufactured by Kyowa Hakko Chemical Co., Ltd.,), and Neodol (trade name, manufactured by Shell Chemicals Japan) and natural alcohols derived from coconut oil, palm oil, palm kernel oil, soy oil, sunflower seed oil, rapeseed oil, beef tallow, and the like. These alcohols can be preferably used in the present invention. Natural alcohols derived from oil-and-fat are particularly preferred from the point of carbon neutrality. In addition of EO and PO to the alcohol, an inert gas may be used together. Any inert gas can be used as long as it does not affect the addition adversely. The addition is performed, for example, at 100 to 200°C, and preferably within the range of 120 to 190°C. A pressure in the addition is 0.1 to 4 MPa, and preferably within the range of 0.2 to 1.5 MPa. In the method of the present invention, to the alcohol are added EO and PO in the presence of a catalyst to give the intended starting compound represented by the formula (1). Examples of the catalyst include sodium hydroxide and potassium hydroxide. An amount of the catalyst is 0.1 mol% to 2 mol% of the alcohol.

The resultant starting compound may be used as it is for the sulfation, or filtered to remove the catalyst therefrom to be used. In filtration to remove the catalyst, a means for filtration may be a filter or the like. Known filters can be used for filtration, including paper filter, cloth filter, two-layer filter of cellulose and polyester, sintered metal filter, and metal mesh filter. Centrifugal separators such as a decanter type centrifugal separator and a centrifugal clarifier may also be used. The resultant starting compound may be contacted with a filtration aid before the filtration or after the centrifugation. Examples of the filtration aid include diatomaceous earth of amorphous silicate containing 80 to 95% of $SiO_2$, aluminium silicate containing about 70% of $SiO_2$, cellulose-based filtration aid, activated charcoal, activated alumina, synthetic zeolite, clay, and clay mineral. These aids may be used alone or in combination of two or more thereof. An amount of the filtration aid used is not specifically limited, but preferably within the range of 0.01 to 5% by mass, and more preferably 0.02 to 3% by mass of the starting compound represented by the formula (1).

An example of production of the starting compound is described below. It does not restrict the production of the starting compound.

(Example of production of the starting compound)

186 g (1.0 mol) of dodecyl alcohol and 0.6 g (0.005 mol) of 48% potassium hydroxide as a catalyst were placed in an autoclave equipped with a stirrer, a temperature controller, and an automatic introduction device. The inner atmosphere of the autoclave was substituted with nitrogen. The autoclave was set to a reduced pressure (1.3 kPa) and 110°C for 0.5 hours for dehydration. To this was added 23.2 g (0.4 mol) of PO at 145°C such that a pressure was 0.1 to 0.4 MPa, and reacted for 1 hour at 145°C. Then, to this was added 66.1 g (1.5 mol) of EO at 155°C such that a pressure was 0.1 to 0.4 MPa, and reacted for 1 hour at 155°C. After the end of EO addition, unreacted EO was removed under reduced pressure. Then, the mixture was cooled to 80°C and 0.5 g (0.005 mol) of 90% lactic acid was added to produce an adduct PO and EO adduct to dodecyl alcohol having 0.4 mol of PO on the average and 1.5 mols of EO on the average.

Examples

The following Examples demonstrate the present invention. Examples are intended to illustrate the present invention and not to limit the present invention.

<Example 1>

[1] Production of sulfate

A production apparatus having a structure as shown in Fig. 1 was used. The production apparatus includes a reaction tube 5 (inner diameter: 14 mmφ, length: 4 m) equipped with first and second jackets 6 and 6' as cooling devices, and a pot 7 for receiving a reaction product. To the production apparatus for sulfate, a starting compound (dodecyl alcohol-PO and EO-adduct (PO 0.4 mol and EO 1.5 mol on average, flow rate: 5.3 kg/Hr)), 4.2% by volume of $SO_3$ gas (flow rate: 200 l/min) diluted in the dry air (air for dilution, flow rate: 70 l/min), and an inert gas (dry air, flow rate: 91 l/min) were fed through inlet ports 2, 4, and 3, respectively. Amounts of the dry air for dilution and the inert gas were regulated such that a gas flow rate at an outlet port of the production apparatus was at a steady level. For the reaction tube 5, parts were set as follows. In the upper first jacket 6 (positioned from 0 m (upper flange part) to 2 m of the reaction tube), water of 50°C was supplied from an inlet to flow. A temperature of water was 50°C at an outlet. In the lower second jacket 6' (positioned from 2 m to 4 m of the reaction tube), water of 16°C was supplied from an inlet to flow. A temperature of water was 17°C at an outlet. A temperature of water at an outlet was measured at a steady state (the same applied below). A sulfated product and an exhaust gas discharged from the production apparatus for sulfate were separated with a gas-liquid separator. The sulfated product was added to an aqueous solution of 1.21% sodium hydroxide under stirring, and adjusted to 10% by mass of active component and a pH of 11 to 13 to give an aqueous solution of the sulfate. In the apparatus, a length of the upstream jacket is 50% of the whole length of the reaction tube, and a length of the downstream jacket is 50%.

[2] Evaluation

The resultant aqueous solution was analyzed for contents of unreacted starting compound and sodium sulfate ($Na_2SO_4$), and evaluated for color (Klett) and odor according to the following methods to judge quality. Results are shown in Table 1. In Table 1, a reaction rate refers to that to the starting compound, and contents of unreacted starting compound and sodium sulfate ($Na_2SO_4$) refer to that in the aqueous solution (the same applied below).

- color (Klett)

An aqueous solution of sodium salt of the sulfated product (active amount: 10% by mass) was measured in a 10 mm cell at 420 nm of wavelength. - odor

Odor of an aqueous solution of sodium salt of the sulfated product (active amount: 10% by mass) was judged by 5 specialists, and ranked by the number of specialists judging that the odor was equal to that of polyoxyethylene alkyl ether sulfate of Control Example in Tables 1 to 9 as follows: "○ (good)" for 4 or more, "△ (fair)" for 2 or 3, and "✕ (not good)" for 1 or less.

<Examples 2 to 9 and Comparative Examples 1 to 9>

Aqueous solutions of sodium salts of the sulfated products (active amount: 10% by mass) were similarly prepared as in Example 1, except that starting compounds and reaction conditions were changed as shown in Tables 2 to 9, and evaluated similarly as in Example 1. Results are shown in Tables 1 to 9.

In each of Example 8 and Comparative Example 8 (Table 8), a reaction tube having an inner diameter of 28 mm and a length of 7 m was used. An upstream first jacket was positioned from 0 m (upper flange part) to 3 m of the reaction tube. A downstream second jacket was positioned from 3 m to 7 m of the reaction tube. In this apparatus, a length of the upstream jacket is 43% of the whole length of the reaction tube, and a length of the downstream jacket is 57%.

In Example 9 and Comparative Example 9 (Table 9), products were measured for 2-MP (2-methyl-2-pentenal) by gas chromatography (GC) instead of color, a reaction rate, and a content of unreacted starting material and a content of sodium sulfate.

In a column of R of the starting compound in Tables, Cn such as C12 refers to an alkyl group having n carbon atoms.

Table 1

| | | Starting compound | | | | | | Reaction condition | | | | | Evaluation of sulfated product (aqueous solution of 10% by mass of active content) | | | | |
| | | Structure in the formula (1) | | | | | | Jacket temperature (°C) | | | | SO$_3$/starting compound (Molar ratio) | Odor | Color (Klett) | Reaction rate (%) | Content of unreacted starting material (% by mass) | Content of Na$_2$SO$_4$ (% by mass) |
| | | R | | (CH$_2$CH$_2$O)p | (C$_n$H$_{2n}$O)m | | (CH$_2$CH$_2$O)k | First (upstream) | | Second (downstream) | | | | | | | |
| | | Composition | Weight ratio | p | n | m | k | Inlet | Outlet | Inlet | Outlet | | | | | | |
| Example | 1-1 | C12 | – | 0 | 3 | 0.4 | 1.5 | 50 | 50 | 16 | 17 | 0.99 | O | 3 | 97 | 0.23 | 0.03 |
| | 1-2 | C12 | – | 0 | 3 | 0.4 | 1.5 | 68 | 70 | 20 | 21 | 0.98 | O | 4 | 98 | 0.12 | 0.02 |
| Comparative example | 1-1 | C12 | – | 0 | 3 | 0.4 | 1.5 | 50 | 50 | 40 | 40 | 0.99 | × | 4 | 97 | 0.20 | 0.02 |
| | 1-2 | C12 | – | 0 | 3 | 2 | 0 | 50 | 51 | 35 | 36 | 0.98 | × | 5 | 97 | 0.25 | 0.03 |
| Reference example | A | C12 | – | 0 | – | 0 | 2 | 52 | 53 | 16 | 16 | 0.99 | Control | 4 | 99 | 0.08 | 0.01 |

EP 2 351 734 B1

Table 2

| | | Starting compound | | | | | | Reaction condition | | | | | Evaluation of sulfated product (aqueous solution of 10% by mass of active content) | | | | |
|---|---|---|---|---|---|---|---|---|---|---|---|---|---|---|---|---|---|
| | | Structure in the formula (1) | | | | | | Jacket temperature (℃) | | | | $SO_3$／starting compound (Molar ratio) | Odor | Color (Klett) | Reaction rate (%) | Content of unreacted starting material (% by mass) | Content of $Na_2SO_4$ (% by mass) |
| | | R | | $(CH_2CH_2O)_p$ | $(C_nH_{2n}O)_m$ | $(CH_2CH_2O)_k$ | | First (upstream) | | Second (downstream) | | | | | | | |
| | | Composition | Weight ratio | p | n | m | k | Inlet | Outlet | Inlet | Outlet | | | | | | |
| Example | 2-1 | C12/C14 | 70/30 | 0 | 3 | 2 | 0 | 50 | 50 | 16 | 17 | 0.99 | ○ | 3 | 98 | 0.18 | 0.02 |
| | 2-2 | C12/C14 | 70/30 | 0 | 3 | 1.5 | 1.5 | 47 | 48 | 17 | 18 | 0.99 | ○ | 4 | 98 | 0.13 | 0.02 |
| | 2-3 | C12/C14 | 70/30 | 0 | 3 | 1.5 | 1.5 | 40 | 40 | 17 | 18 | 0.99 | ○ | 3 | 97 | 0.22 | 0.02 |
| | 2-4 | C12/C14 | 70/30 | 0 | 3 | 1.5 | 1.5 | 51 | 50 | 17 | 18 | 0.97 | ○ | 4 | 97 | 0.20 | 0.01 |
| | 2-5 | C12/C14 | 70/30 | 0 | 3 | 0.7 | 1.5 | 40 | 41 | 16 | 18 | 0.99 | ○ | 3 | 96 | 0.28 | 0.02 |
| | 2-6 | C12/C14 | 70/30 | 0 | 3 | 0.4 | 8 | 40 | 40 | 20 | 21 | 1.01 | ○ | 1 | 99 | 0.10 | 0.05 |
| | 2-7 | C12/C14 | 70/30 | 0 | 3 | 0.4 | 1.5 | 49 | 50 | 16 | 17 | 0.99 | ○ | 3 | 97 | 0.23 | 0.03 |
| Comparative example | 2-1 | C12/C14 | 70/30 | 0 | 3 | 0.4 | 1.5 | 20 | 18 | 20 | 18 | 0.99 | × | 4 | 97 | 0.20 | 0.06 |
| | 2-2 | C12/C14 | 70/30 | 0 | 3 | 0.4 | 1.5 | 50 | 50 | 48 | 48 | 0.99 | × | 5 | 99 | 0.08 | 0.01 |
| Reference example | 1 | C12/C14 | 70/30 | 0 | − | 0 | 2 | 18 | 22 | 17 | 17 | 0.99 | Control | 10 | 98 | 0.13 | 0.02 |
| | 2 | C12/C14 | 70/30 | 0 | − | 0 | 2 | 52 | 53 | 16 | 16 | 0.99 | ○ | 4 | 99 | 0.08 | 0.01 |
| | 3 | C12/C14 | 70/30 | 0 | − | 0 | 2 | 48 | 49 | 52 | 51 | 0.99 | ○ | 5 | 99 | 0.08 | 0.01 |

EP 2 351 734 B1

Table 3

| | | Starting compound | | | | | Reaction condition | | | | | Evaluation of sulfated product (aqueous solution of 10% by mass of active content) | | | | |
| | | Structure in the formula (1) | | | | | Jacket temperature (°C) | | | | SO₃／Starting compound (Molar ratio) | Odor | color (Klett) | Reactio n rate (%) | Content of unreacted starting material (% by mass) | Content of Na₂SO₄ (% by mass) |
| | | R | | $(CH_2CH_2O)_p$ | $(C_nH_{2n}O)_m$ | $(CH_2CH_2O)_k$ | First (upstream) | | Second (downstream) | | | | | | | |
| | | Composition | Weight ratio | p | n | m | k | Inlet | Outlet | Inlet | Outlet | | | | | | |
| Example | 3 | C18 | – | 0 | 3 | 3 | 0 | 60 | 61 | 30 | 30 | 0.99 | ○ | 6 | 96 | 0.29 | 0.01 |
| Comparative example | 3 | C18 | – | 0 | 3 | 3 | 0 | 60 | 62 | 50 | 50 | 0.99 | × | 8 | 96 | 0.27 | 0.04 |
| Reference example | B | C18 | — | 3 | – | 0 | 0 | 60 | 61 | 50 | 50 | 0.99 | Control | 6 | 96 | 0.28 | 0.01 |

Table 4

| | | Starting compound | | | | | | Reaction condition | | | | | Evaluation of sulfated product (aqueous solution of 10% by mass of active content) | | | | |
|---|---|---|---|---|---|---|---|---|---|---|---|---|---|---|---|---|---|
| | | Structure in the formula(1) | | | | | | Jacket temperature (°C) | | | | SO$_3$／starting compound (molar ratio) | Odor | Color (Klett) | Reaction rate (%) | Content of unreacted starting material (% by mass) | Content of Na$_2$SO$_4$(% by mass) |
| | | R | | $(CH_2CH_2O)_p$ | $(C_nH_{2n}O)_m$ | | $(CH_2CH_2O)_k$ | First (upstream) | | Second (downstream) | | | | | | | |
| | | Composition | Weight ratio | p | n | m | k | Inlet | Outlet | Inlet | Outlet | | | | | | |
| Example | 4-1 | C12 | − | 0 | 3 | 0.4 | 1.5 | 50 | 50 | 16 | 17 | 0.99 | ○ | 3 | 97 | 0.23 | 0.03 |
| | 4-2 | C12/C14 | 70/30 | 0 | 3 | 1.5 | 1.5 | 47 | 48 | 17 | 18 | 0.99 | ○ | 4 | 98 | 0.13 | 0.02 |
| | 4-3 | C12/C14 | 70/30 | 0 | 3 | 0.7 | 1.5 | 40 | 41 | 16 | 18 | 0.99 | ○ | 3 | 96 | 0.28 | 0.02 |
| | 4-4 | C12/C14 | 70/30 | 0 | 3 | 1.5 | 1.5 | 40 | 40 | 17 | 18 | 0.99 | ○ | 3 | 97 | 0.22 | 0.02 |
| | 4-5 | C12/C14 | 70/30 | 0 | 3 | 0.4 | 1.5 | 49 | 50 | 16 | 17 | 0.99 | ○ | 3 | 97 | 0.23 | 0.03 |
| | 4-6 | C12/C14 | 70/30 | 0 | 3 | 1.5 | 1.5 | 51 | 50 | 17 | 18 | 0.97 | ○ | 4 | 97 | 0.20 | 0.01 |
| Comparative example | 4-1 | C12/C14 | 70/30 | 0 | 3 | 0.4 | 1.5 | 20 | 18 | 20 | 18 | 0.99 | × | 4 | 97 | 0.20 | 0.06 |
| | 4-2 | C12/C14 | 70/30 | 0 | 3 | 0.4 | 1.5 | 50 | 50 | 48 | 48 | 0.99 | × | 5 | 99 | 0.08 | 0.01 |
| | 4-3 | C12 | − | 0 | 3 | 0.4 | 1.5 | 50 | 50 | 40 | 40 | 0.99 | × | 4 | 97 | 0.20 | 0.02 |
| Control example | 1 | C12/C14 | 70/30 | 0 | − | 0 | 2 | 18 | 22 | 17 | 17 | 0.99 | Control | 10 | 98 | 0.13 | 0.02 |
| | 2 | C12/C14 | 70/30 | 0 | − | 0 | 2 | 52 | 53 | 16 | 16 | 0.99 | ○ | 4 | 99 | 0.08 | 0.01 |
| | 3 | C12/C14 | 70/30 | 0 | − | 0 | 2 | 48 | 49 | 52 | 51 | 0.99 | ○ | 5 | 99 | 0.08 | 0.01 |

Table 5a

| | | Starting compound | | | | | Reaction condition | | | | | Evaluation of sulfated product (aqueous solution of 10% by mass of active content) | | | | |
|---|---|---|---|---|---|---|---|---|---|---|---|---|---|---|---|---|---|
| | | Structure in the formula(1) | | | | | Jacket temperature (℃) | | | | $SO_3$/Starting compound (molar ratio) | Odor | Color (Klett) | Reaction rate (%) | Content of unreacted starting material (% by mass) | Content of $Na_2SO_4$ (% by mass) |
| | | R | | $(CH_2CH_2O)p$ | $(C_nH_{2n}O)m$ | $(CH_2CH_2O)k$ | First (upstream) | | Second (downstream) | | | | | | | |
| | | Composition | Weight ratio | p | n | m | k | Inlet | Outlet | Inlet | Outlet | | | | | | |
| Example | 5-1 | C10 | – | 0 | 3 | 0.7 | 0 | 40 | 41 | 9 | 11 | 0.99 | ○ | 4 | 98 | 0.17 | 0.03 |
| Reference example | C | C10 | – | 1 | – | 0 | 0 | 40 | 41 | 9 | 11 | 0.99 | Control | 4 | 98 | 0.16 | 0.02 |

EP 2 351 734 B1

Table 5b

| | | Starting compound | | | | | | Reaction condition | | | | | Evaluation of sulfated product (aqueous solution of 10% by mass of active content) | | | | |
|---|---|---|---|---|---|---|---|---|---|---|---|---|---|---|---|---|---|
| | | Structure in the formula (1) | | | | | | Jacket temperature (°C) | | | | SO$_3$／Starting compound (Molar ratio) | Odor | Color (klett) | Reaction rate (%) | Content of unreacted starting material (% by mass) | Content of Na$_2$SO$_4$ (% by mass) |
| | | R | | (CH$_2$CH$_2$O)p | (C$_n$H$_{2n}$O)m | | (CH$_2$CH$_2$O)k | First (upstream) | | Second (downstream) | | | | | | | |
| | | Composition | Weight ratio | p | n | m | k | Inlet | Outlet | Inlet | Outlet | | | | | | |
| Example | 5-2 | C12/C14 | 70/30 | 0 | 3 | 2 | 0 | 50 | 50 | 16 | 17 | 0.99 | ○ | 3 | 98 | 0.18 | 0.02 |
| Comparative example | 5-1 | C12/C14 | 70/30 | 0 | 3 | 2 | 0 | 50 | 51 | 35 | 36 | 0.99 | × | 5 | 97 | 0.25 | 0.03 |
| Reference example | 1 | C12/C14 | 70/30 | 0 | – | 0 | 2 | 18 | 22 | 17 | 17 | 0.99 | Control | 10 | 98 | 0.13 | 0.02 |

EP 2 351 734 B1

15

Table 5c

| | | Starting compound | | | | | Reaction condition | | | | | Evaluation of sulfated product (aqueous solution of 10% by mass of active content) | | | | |
|---|---|---|---|---|---|---|---|---|---|---|---|---|---|---|---|---|
| | | Structure in the formula (1) | | | | | Jacket temperature (℃) | | | | SO₃／Starting compound (Molar ratio) | | | | | |
| | | R | | $(CH_2CH_2O)p$ | $(C_nH_{2n}O)m$ | $(CH_2CH_2O)k$ | First (upstream) | | Second (downstream) | | | Odor | Color (klett) | Reaction rate (%) | Content of unreacted starting material (% by mass) | Content of $Na_2SO_4$ (% by mass) |
| | | Composition | Weight ratio | p | n | m | k | Inlet | Outlet | Inlet | Outlet | | | | | |
| Example | 5-3 | C16 | – | 0 | 3 | 2 | 0 | 55 | 56 | 25 | 28 | 0.99 | ○ | 5 | 97 | 0.26 | 0.01 |
| Example | 5-4 | C18 | – | 0 | 3 | 3 | 0 | 60 | 61 | 30 | 30 | 0.99 | ○ | 6 | 96 | 0.29 | 0.01 |
| Comparative example | 5-2 | C18 | – | 0 | 3 | 3 | 0 | 60 | 62 | 50 | 50 | 0.99 | × | 8 | 96 | 0.27 | 0.04 |
| Reference example | B | C18 | – | 3 | – | 0 | 0 | 60 | 61 | 50 | 50 | 0.99 | Control | 6 | 98 | 0.28 | 0.01 |

Table 6

| | | Starting compound | | | | | Reaction condition | | | | | Evaluation of sulfated product (aqueous solution of 10% by mass of active content) | | | | |
|---|---|---|---|---|---|---|---|---|---|---|---|---|---|---|---|---|
| | | Structure in the formula(1) | | | | | Jacket temperature ($^{\circ}$C) | | | | $SO_3$/Starting compound (Molar ratio) | Odor | Color (klett) | Reaction rate (%) | Content of unreacted starting material (% by mass) | Content of $Na_2SO_4$ (% by mass) |
| | | R | | $(CH_2CH_2O)_p$ | $(C_nH_{2n}O)_m$ | | $(CH_2CH_2O)_k$ | First (upstream) | | Second (downstream) | | | | | | |
| | | Composition | Weight ratio | p | n | m | k | Inlet | Outlet | Inlet | Outlet | | | | | | |
| Example | 6-1 | C12 | – | 0 | 3 | 0.4 | 1.5 | 50 | 50 | 16 | 17 | 0.99 | ○ | 3 | 97 | 0.23 | 0.03 |
| | 6-2 | C12/C14 | 70/30 | 0 | 3 | 0.4 | 1.5 | 49 | 50 | 16 | 17 | 0.99 | ○ | 3 | 97 | 0.23 | 0.03 |
| | 6-3 | C12/C14 | 70/30 | 0 | 3 | 0.4 | 8 | 40 | 40 | 20 | 21 | 1.01 | ○ | 1 | 99 | 0.10 | 0.05 |
| Comparative example | 6-1 | C12/C14 | 70/30 | 0 | 3 | 0.4 | 1.5 | 20 | 18 | 20 | 18 | 0.99 | × | 4 | 97 | 0.20 | 0.06 |
| | 6-2 | C12 | – | 0 | 3 | 0.4 | 1.5 | 50 | 50 | 40 | 40 | 0.99 | × | 4 | 97 | 0.20 | 0.02 |
| | 6-3 | C12/C14 | 70/30 | 0 | 3 | 0.4 | 1.5 | 50 | 50 | 48 | 48 | 0.99 | × | 5 | 99 | 0.08 | 0.01 |
| Reference example | 1 | C12/C14 | 70/30 | 0 | – | 0 | 2 | 18 | 22 | 17 | 17 | 0.99 | Control | 10 | 98 | 0.13 | 0.02 |
| | 2 | C12/C14 | 70/30 | 0 | – | 0 | 2 | 52 | 53 | 16 | 16 | 0.99 | ○ | 4 | 99 | 0.08 | 0.01 |
| | 3 | C12/C14 | 70/30 | 0 | – | 0 | 2 | 48 | 49 | 52 | 51 | 0.99 | ○ | 5 | 99 | 0.08 | 0.01 |

EP 2 351 734 B1

Table 7

| | | Starting compound | | | | | Reaction condition | | | | | Evaluation of sulfated product (aqueous solution of 10% by mass of active content) | | | | |
|---|---|---|---|---|---|---|---|---|---|---|---|---|---|---|---|---|---|
| | | Structure in the formula(1) | | | | | Jacket temperature (℃) | | | | $SO_3$／Starting compound (Molar ratio) | Odor | Color (klett) | Reaction rate (%) | Content of unreacted starting material (% by mass) | Content of $Na_2SO_4$ (% by mass) |
| | | R | | $(CH_2CH_2O)_p$ | $(C_nH_{2n}O)_m$ | $(CH_2CH_2O)_k$ | First (upstream) | | Second (downstream) | | | | | | | |
| | | Composition | Weight ratio | p | n | m | k | Inlet | Outlet | Inlet | Outlet | | | | | | |
| Example | 7 | C12/C14 | 70/30 | 7 | 3 | 2 | 3 | 50 | 50 | 15 | 16 | 0.99 | ○ | 7 | 98 | 0.15 | 0.03 |
| Comparative example | 7 | C12/C14 | 70/30 | 7 | 3 | 2 | 3 | 50 | 50 | 48 | 48 | 0.99 | × | 9 | 98 | 0.11 | 0.01 |
| Reference example | D | C12/C14 | 70/30 | 12 | − | 0 | 0 | 50 | 50 | 48 | 48 | 0.99 | Control | 9 | 98 | 0.12 | 0.02 |

Table 8

| | | Starting compound | | | | | | Reaction condition | | | | | Evaluation of sulfated product (aqueous solution of 10% by mass of active content) | | | | |
|---|---|---|---|---|---|---|---|---|---|---|---|---|---|---|---|---|---|
| | | Structure in the formula(1) | | | | | | Jacket temperature (°C) | | | | SO$_3$／Starting compound (Molar ratio) | Odor | Color (klett) | Reaction rate (%) | Content of unreacted starting material (% by mass) | Content of Na$_2$SO$_4$ (% by mass) |
| | | R | | (CH$_2$CH$_2$O)p | (C$_n$H$_{2n}$O)m | (CH$_2$CH$_2$O)k | | First (upstream) | | Second (downstream) | | | | | | | |
| | | Composition | Weight ratio | p | n | m | k | Inlet | Outlet | Inlet | Outlet | | | | | | |
| Example | 8 | C12/C14 | 70/30 | 0 | 3 | 2 | 3 | 49 | 52 | 16 | 18 | 0.99 | ○ | 8 | 98 | 0.15 | 0.03 |
| Comparative example | 8 | C12/14 | 70/30 | 0 | 3 | 2 | 3 | 70 | 71 | 15 | 16 | 0.99 | × | 9 | 98 | 0.11 | 0.01 |
| Reference example | 1 | C12/C14 | 70/30 | 0 | --- | 0 | 2 | 18 | 22 | 17 | 17 | 0.99 | Control | 10 | 98 | 0.13 | 0.02 |

EP 2 351 734 B1

Table 9

| | | | Starting compound | | | | | Reaction condition | | | | | Evaluation of sulfated product (aqueous solution of 10% by mass of active content) | |
| | | | Structure in the formula(1) | | | | | Jacket temperature (°C) | | | | SO₃／Starting compound (Molar ratio) | Odor | 2-MP (GC-area%) |
| | | | R | | $(CH_2CH_2O)_p$ | $(C_nH_{2n}O)_m$ | $(CH_2CH_2O)_k$ | First (upstream) | | Second (downstream) | | | | |
| | | | Composition | Weight ratio | p | n | m | k | Inlet | Outlet | Inlet | Outlet | | | |
| Example | 9-1 | C12/C14 | 70/30 | 0 | 3 | 1.5 | 1.5 | 47 | 48 | 17 | 18 | 0.99 | ○ | 0.1 | | |
| | 9-2 | C12/C14 | 70/30 | 0 | 3 | 2 | 0 | 50 | 50 | 16 | 17 | 0.99 | ○ | 0.3 | | |
| | 9-3 | C12/C14 | 70/30 | 0 | 3 | 0.7 | 1.5 | 40 | 41 | 16 | 18 | 0.99 | ○ | 0.2 | | |
| | 9-4 | C12/C14 | 70/30 | 0 | 3 | 1.5 | 1.5 | 40 | 40 | 17 | 18 | 0.99 | ○ | 0.1 | | |
| | 9-5 | C12/C14 | 70/30 | 0 | 3 | 0.4 | 1.5 | 49 | 50 | 16 | 17 | 0.99 | ○ | 0.1 | | |
| | 9-6 | C12/C14 | 70/30 | 0 | 3 | 1.5 | 1.5 | 51 | 50 | 17 | 18 | 0.97 | ○ | 0.1 | | |
| Comparative example | 9-1 | C12/C14 | 70/30 | 0 | 3 | 0.4 | 1.5 | 20 | 18 | 20 | 18 | 0.99 | × | 2.1 | | |
| | 9-2 | C12/C14 | 70/30 | 0 | 3 | 0.4 | 1.5 | 50 | 50 | 48 | 48 | 0.99 | × | 2.3 | | |
| Reference example | 1 | C12/C14 | 70/30 | 0 | − | 0 | 2 | 18 | 22 | 17 | 17 | 0.99 | Control | − | | |

**Claims**

1. A method for producing a sulfate represented by the formula (2), comprising steps of:

feeding a compound represented by the formula (1) (starting compound) as a flow running down along an inner wall of a reaction tube to form a thin film of the starting compound on the inner wall of the reaction tube, the tube being provided in a jacket system comprising two or more temperature-controlled jackets in which a temperature-controlling fluid can flow, and together feeding $SO_3$ gas into the reaction tube to react the starting compound with the $SO_3$ gas to obtain a sulfated product; and
neutralizing the resultant sulfated product,
wherein the two or more jackets include an upstream jacket placed on an upstream part and a downstream jacket placed on a downstream part in a direction of the flow of the starting compound,
an exit temperature of the temperature-controlling fluid flowing in the upstream jacket is not less than 35°C and not more than 70°C,
an exit temperature of the temperature-controlling fluid flowing in the downstream jacket is not less than 10°C and not more than 30°C,
a length of the upstream jacket is 40 to 60% of the whole length of the reaction tube, and
a length of the downstream jacket is 60 to 40% of the whole length of the reaction tube:

$$R\text{-}O\text{-}(CH_2CH_2O)_p\text{-}(C_nH_{2n}C)_m\text{-}(CH_2CH_2O)_k\text{-}H \qquad (1)$$

where R represents a hydrocarbon group having 8 to 22 carbon atoms; p and k each represents a number, a summation average of p and k being not less than 0 and not more than 40; n represents a number of 3 to 4; and m represents a number of more than 0 and not more than 5 on average,

$$R\text{-}O\text{-}(CH_2CH_2O)p\text{-}(C_nH_{2n}C)_m\text{-}(CH_2CH_2O)_k\text{-}SO_3M \qquad (2)$$

where R represents a hydrocarbon group having 8 to 22 carbon atoms; p and k each represents a number, a summation average of p and k being not less than 0 and not more than 40; n represents a number of 3 to 4; m represents a number of more than 0 and not more than 5 on average; M represents an alkaline metal ion, an alkaline earth metal ion, an ammonium ion, or mono-, di-, or trialkanolammonium ion having an alkanol group having 2 to 3 carbon atoms.

2. The method for producing a sulfate according to claim 1, wherein a reaction molar ratio of the starting compound to $SO_3$ gas, $SO_3$/starting compound, is 0.90 to 1.09.

3. The method for producing a sulfate according to claim 1 or 2, wherein an inert gas is fed as flowing between the thin film of the starting compound and the $SO_3$ gas.

4. The method for producing a sulfate according to any one of claims 1 to 3, wherein an inner diameter of the reaction tube is 8 to 80 mm.

5. The method for producing a sulfate according to any one of claims 1 to 4, wherein a length of the reaction tube is 1 to 20 m.

**Patentansprüche**

1. Verfahren zur Herstellung eines Sulfats der Formel (2), umfassend die Schritte:

Zufuhr einer Verbindung der Formel (1) (Startverbindung) als Durchfluss, der entlang einer inneren Wand einer Reaktionsröhre hinab fließt, um einen dünnen Film der Startverbindung auf der inneren Wand der Reaktionsröhre zu bilden, wobei die Röhre in einem Ummantelungssystem bereitgestellt ist, das zwei oder mehrere temperaturgesteuerte Ummantelungen umfasst, in denen ein Temperatursteuerungsfluid fließen kann, und gemeinsames Zuführen von $SO_3$-Gas in die Reaktionsröhre, um die Startverbindung mit dem $SO_3$-Gas umzusetzen, um ein sulfatiertes Produkt zu erhalten; und
Neutralisieren des resultierenden sulfatierten Produkts,
worin die zwei oder mehreren Ummantelungen eine vorgelagerte Ummantelung, platziert an einem vorgelagerten Teil, und eine nachgelagerte Ummantelung, platziert an einem nachgelagerten Teil in Bezug auf die Flussrichtung der Startverbindung, einschließen,

eine Ausflusstemperatur des Temperatursteuerungsfluids, das in der vorgelagerten Ummantelung fließt, nicht weniger als 35°C und nicht mehr als 70°C ist,

eine Ausflusstemperatur des Temperatursteuerungsfluids, das in der nachgelagerten Ummantelung fließt, nicht weniger als 10°C und nicht mehr als 30°C ist,

eine Länge der vorgelagerten Ummantelung 40 bis 60 % der Gesamtlänge der Reaktionsröhre ist, und

eine Länge der nachgelagerten Ummantelung 60 bis 40 % der gesamten Länge der Reaktionsröhre ist:

$$R\text{-}O\text{-}(CH_2CH_2O)_p\text{-}(C_nH_{2n}C)_m\text{-}CH_2CH_2O)_k\text{-}H$$

worin R eine Kohlenwasserstoffgruppe mit 8 bis 22 Kohlenstoffatomen darstellt; p und k jeweils eine Zahl darstellen, ein Summenmittelwert von p und k nicht weniger als 0 und nicht mehr als 40 ist; n eine Zahl von 3 bis 4 darstellt; und m eine Zahl von mehr als 0 und nicht mehr als 5 im Mittel darstellt,

$$R\text{-}O\text{-}(CH_2CH_2O)p\text{-}(C_nH_{2n}C)_m\text{-}CH_2CH_2O)_k\text{-}SO_3M$$

worin R eine Kohlenwasserstoffgruppe mit 8 bis 22 Kohlenstoffatomen darstellt; p und k jeweils eine Zahl darstellen, ein Summenmittelwert von p und k nicht weniger als 0 und nicht mehr als 40 ist; n eine Zahl von 3 bis 4 darstellt; m eine Zahl von mehr als 0 und nicht mehr als 5 im Mittel darstellt; M ein Alkaliion, ein Erdalkalimetallion, ein Ammoniumion oder ein Mono-, Di-, oder Trialkanolammoniumion, das eine Alkanolgruppe mit 2 bis 3 Kohlenstoffatomen aufweist, darstellt.

2. Verfahren zur Herstellung eines Sulfats gemäß Anspruch 1, worin ein Reaktionsmolverhältnis der Startverbindung zum $SO_3$-Gas, $SO_3$/Startverbindung 0,90 bis 1,09 ist.

3. Verfahren zur Herstellung eines Sulfats gemäß Anspruch 1 oder 2, worin ein Inertgas als Fluss zwischen dem Dünnfilm der Startverbindung und dem $SO_3$-Gas zugeführt wird.

4. Verfahren zur Herstellung eines Sulfats gemäß irgendeinemder Ansprüche 1 bis 3, worin ein Innendurchmesser der Reaktionsröhre 8 bis 80 mm ist.

5. Verfahren zur Herstellung eines Sulfats gemäß irgendeinem der Ansprüche 1 bis 4, worin eine Länge der Reaktionsröhre 1 bis 20 m ist.

**Revendications**

1. Procédé pour produire un sulfate représenté par la formule (2), comprenant les étapes consistant à :

amener un composé représenté par la formule (1) (composé de départ) en tant qu'un flux courant le long d'une paroi intérieure d'un tube de réaction pour former un film mince du composé de départ sur la paroi intérieure du tube de réaction, le tube étant disposé dans un système de chemises comprenant deux chemises ou plus commandées en température dans lesquelles un fluide commandant la température peut s'écouler, et amener concurremment du gaz $SO_3$ dans le tube de réaction pour faire réagir le composé de départ avec le gaz $SO_3$ pour obtenir un produit sulfaté ; et

neutraliser le produit sulfaté résultant,

dans lequel les deux ou plusieurs chemises incluent une chemise amont placée sur une partie amont et une chemise aval placée sur une partie aval dans une direction du flux du composé de départ,

une température de sortie du fluide commandant la température s'écoulant dans la chemise amont n'est pas inférieure à 35 °C et pas supérieure à 70 °C,

une température de sortie du fluide commandant la température s'écoulant dans la chemise aval n'est pas inférieure à 10 °C et pas supérieure à 30 °C,

une longueur de la chemise amont est de 40 à 60 % de la longueur entière du tube de réaction, et

une longueur de la chemise aval est de 60 à 40 % de la longueur entière du tube de réaction :

$$R\text{-}O\text{-}(CH_2CH_2O)_p\text{-}(C_nH_{2n}C)_m\text{-}(CH_2CH_2O)_k\text{-}H \qquad (1)$$

où R représente un groupe hydrocarbure ayant de 8 à 22 atomes de carbone ; p et k représentent chacun un nombre, une moyenne de sommation de p et k étant non inférieure à 0 et non supérieure à 40 ; n représente un nombre de 3 à 4 ; et m représente un nombre supérieur à 0 et non supérieur à 5 en moyenne,

$$R-O-(CH_2CH_2O)p-(C_nH_{2n}C)_{m-}(CH_2CH_2O)_k-SO_3M \qquad (2)$$

où R représente un groupe hydrocarbure ayant de 8 à 22 atomes de carbone ; p et k représentent chacun un nombre, une moyenne de sommation de p et k étant non inférieure à 0 et non supérieure à 40 ; n représente un nombre de 3 à 4 ; m représente un nombre supérieur à 0 et non supérieur à 5 en moyenne ; M représente un ion de métal alcalin, un ion de métal alcalino-terreux, un ion d'ammonium, ou un ion de mono-, di-, ou trialcanolammonium ayant un groupe alcanol ayant 2 à 3 atomes de carbone.

2.  Procédé pour produire un sulfate selon la revendication 1, dans lequel un rapport molaire de réaction du composé de départ sur du gaz $SO_3$, $SO_3$/composé de départ, est de 0,90 à 1,09.

3.  Procédé pour produire un sulfate selon la revendication 1 ou 2, dans lequel un gaz inerte est amené comme s'écoulant entre le film mince du composé de départ et le gaz $SO_3$.

4.  Procédé pour produire un sulfate selon l'une quelconque des revendications 1 à 3, dans lequel un diamètre intérieur du tube de réaction est de 8 à 80 mm.

5.  Procédé pour produire un sulfate selon l'une quelconque des revendications 1 à 4, dans lequel une longueur du tube de réaction est de 1 à 20 m.

Fig. 1

Fig.2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9048409 B
- JP 3041130 B
- JP 157464 A
- JP 00038374 A
- JP 1315061 A
- JP 995403 B

- JP 001151746 A
- JP 001187776 A
- US 017875 B6
- US A3931273 A
- JP 001181215 A
- JP 002779 B

**Non-patent literature cited in the description**

- Sulfonation and Sulfation. **CHEMITHON, BALL-ESTRA et al.** Kirk Othmer, Encyclopedia of Chemical Technology. Wiley, 1983, vol. 22, 28-45
- **W. H. DE GROOT.** Sulphonation Technology in the Detergent Industry. Kluwer Academic Publishers, 1991

- Detergent Manufacturing Including Zeolite Builders and Other New Materials. Chemical Technology Review No. 128. Noyes Data Corp, 1979, 273-310